# EUROPÄISCHE PATENTSCHRIFT

(11) **EP 1 062 939 B1**
(45) Veröffentlichungstag und Bekanntmachung des Hinweises auf die Patenterteilung: **03.01.2001**
(21) Anmeldenummer: 99124991.3
(22) Anmeldetag: 15.12.1999
(51) Int. Cl.: A61K 7/13, A61K 7/00

(54) **Verfahren zur Färbung von Haar und dafür geeignete Zubereitungen**
Hair dyeing process and suitable compositions
Procede de teinture de cheveux et compositions de teinture

(30) Priorität: 02.10.1999 DE 19947543
(43) Veröffentlichungstag der Anmeldung: 27.12.2000
(73) Patentinhaber: Basler Haar-Kosmetik GmbH, 74321 Bietigheim-Bissingen (DE)
(72) Erfinder: Hermann Basler, 74321 Bietigheim-Bissingen (DE)
(74) Vertreter: Hase, Christian, Dr.

(56) Entgegenhaltungen:
- DE-A- 2 532 036
- GB-A- 1 563 674
- US-A- 3 920 384
- US-A- 4 529 404

## Beschreibung

Die im folgenden beschriebene Erfindung liegt auf den Gebieten der Färberei und der Kosmetik und betrifft Verfahren und Mittel zur dauerhaften Färbung von keratinhaltigen Fasern wie Wolle und Pelz, insbesondere aber zur dauerhaften Färbung von menschlichem Haar.

Der Wunsch, die eigene Haarfarbe zu verändern, ist weit verbreitet. Vor allem Menschen, deren Haar ganz oder teilweise ergraut ist, haben oft den Wunsch, diese Erscheinung rückgängig zu machen. Dementsprechend sind zahlreiche chemische Verfahren entwickelt worden, um diesen Zweck zu erreichen. So kennt man Haarfärbemittel, mit denen sich temporäre, semipermanente und permanente Färbungen auf dem Haar in vielen verschiedenen Farbtönen erzeugen lassen. Vor allem die Mittel zur dauerhaften Färbung der Haare haben weite Verbreitung gefunden. Die Färbung basiert hier in der Regel auf oxidativer Entwicklung der Farbe aus Farbstoffvorprodukten, die mit den Haarfärbemitteln auf das Haar aufgebracht und dort mit Hilfe zusätzlich aufgebrachter chemischer Oxidationsmittel in die eigentlichen Farbstoffe übergeführt werden. Als Oxidationsmittel wird meist Wasserstoffperoxid verwendet, doch ist auch der Einsatz anderer anorganischer Peroxide und Oxidationsmittel gegebenenfalls zusammen mit Oxidationskatalysatoren beschrieben worden. Nachteilig bei diesem Verfahren der Haarfärbung sind insbesondere die durch die Anwendung des Oxidationsmittels unvermeidliche Haarschädigung und die zum Teil nicht unbedenklichen toxikologischen und dermatologischen Eigenschaften vieler als Haarfarbstoffvorprodukte vorgeschlagener Verbindungen.

Neben den Haarfärbemitteln, die an den Einsatz chemischer Oxidationsmittel gebunden sind, kennt man auch die sogenannten selbstoxidierenden Haarfärbemittel, das sind Zubereitungen von bestimmten leicht oxidierbaren Haarfarbstoffvorprodukten, die im Kontakt mit dem Luftsauerstoff in ausreichend kurzer Zeit permanente Haarfärbungen entwickeln. Derartige Haarfärbemittel werden beispielsweise in der deutschen Offenlegungsschrift 2532036 und in der darin zitierten älteren Literatur beschrieben.

Obwohl bei Anwendung der autoxidierenden Haarfärbemittel durch den Verzicht auf ein chemisches Oxidationsmittel Haarschädigungen weitgehend vermieden werden, haben Haarfärbemittel dieser Art bis in jüngste Zeit wenig Verbreitung gefunden. Die Gründe dafür waren verschiedener Art: So waren nur wenige der zahlreichen bekannten Haarfarbstoffvorprodukte ausreichend schnell mit Luftsauerstoff oxidierbar, um bei vertretbaren Konzentrationen in ausreichend kurzer Zeit die gewünschten Farbtiefen zu erreichen. Die ausreichend schnell oxidierbaren Farbstoffvorprodukte lieferten in vielen Fällen stumpfe und graue Farbtöne oder solche Farben, die sich nachträglich langsam unter Auftreten unerwünschter Gelb- oder Rotreflexe veränderten. Die Schwierigkeiten, geeignete Farbstoffvorprodukte für autoxidierende Haarfärbemittel zu finden, wurden noch dadurch verstärkt, daß nicht nur klare und natürlich wirkende Farbtöne bevorzugt wurden, sondern diese Farbtöne darüber hinaus auch lichtbeständig, auswaschbeständig und beständig gegen chloriertes Badewasser sein sollten. Darüber hinaus wurde von den Färbemitteln ein gutes Egalisiervermögen erwartet, d.h. sie sollten stark strapaziertes Haar möglichst in gleicher Weise anfärben wie frisch nachgewachsenes Haar.

Im Bemühen, autoxidierende Haarfärbemittel zu finden, die allen Anforderungen der Praxis genügen, ist bis in jüngste Zeit immer wieder an Neuentwicklungen gearbeitet worden. Beispielhaft sei hier nur auf die europäische Patentanmeldung 414585 und die deutsche Offenlegungsschrift 19717223 verwiesen. Obwohl inzwischen auch erste Haarfärbemittel, die allein durch Luftsauerstoff entwickelt werden, auf dem Markt erschienen sind, kann auch heute noch nicht davon gesprochen werden, daß diese Mittel alle Anforderungen, die sich in der Praxis stellen, in ausreichendem Maße erfüllen. Sie sind vielfach nur für bestimmte Haarsorten geeignet und liefern auf ungeeignetem Haar Fehlfärbungen, führen oft zu unerwünscht dunklen Färbungen, so daß die Herstellung von hellen, blonden Farbtönen Schwierigkeiten bereitet, oder lassen in der Farbbeständigkeit gegen Licht, Auswaschen und Ausbleichen zu wünschen übrig. Es bestand daher nach wie vor ein Bedarf an autoxidablen Haarfärbemitteln mit verbesserten Eigenschaften.

Auch den Entwicklungen, die zur vorliegenden Erfindung geführt haben, lag die Aufgabe zugrunde, autoxidable Haarfärbemittel zu finden, die den Bedürfnissen der Praxis besser angepaßt sind als die bereits bekannten Mittel. Überraschenderweise wurde gefunden, daß dieses Ziel in sehr einfacher Weise durch Kombination bestimmter lange bekannter Oxidationsfarbstoffvorprodukte erreicht werden kann.

Gegenstand der Erfindung ist ein Verfahren zur Färbung von Haar, bei dem eine wäßrige Zubereitung von Haarfarbstoffvorprodukten ohne Zusatz von Oxidationsmittel mit dem Haar in Kontakt gebracht wird und sich die Farbe durch Einwirkung des Sauerstoffs der Luft entwickelt, das dadurch gekennzeichnet ist, daß die wäßrige Zubereitung als Farbstoffvorprodukte 1,2,4-Trihydroxybenzol, N,N-Bis-(2-hydroyxyethyl)-p-phenylendiamin und 2,4,5,6-Tetraaminopyrimidin enthält. In einer besonderen Ausführungsform ist darüber hinaus als weiteres Farbstoffvorprodukt 2,7-Dihydroxynaphthalin enthalten.

Das neue Verfahren erlaubt es überraschenderweise, mit niedrigen Konzentrationen an toxikologisch und dermatologisch weitgehend unbedenklichen Haarfarbstoffvorprodukten klare und helle Farbtöne auf grauem Haar zu erhalten, die von hellblond bis mittelbraun reichen. Die Entwicklung der Farbe erfolgt nach dem Auftragen der wäßrigen Zubereitung auf das Haar im Kontakt mit dem Luftsauerstoff ohne weiteres Zutun schnell und zuverlässig, so daß überschüssige Zubereitung bereits nach kurzer Zeit mit Wasser oder einer Shampoo-Lösung aus dem Haar ausgespült werden kann. Die mit dem neuen Verfahren erzielbaren Haarfärbungen zeichnen sich durch eine hervorragende Beständigkeit gegen Sonnenlicht, gegen Auswaschen und gegen chlorhaltiges Badewasser aus. Eine nachträgliche Veränderung des Farbtons ist nicht zu beobachten.

Die im erfindungsgemäßen Verfahren verwendete wäßrige Zubereitung der Farbstoffvorprodukte kann verschiedene Formen haben. Prinzipiell eignen sich dünnflüssige wäßrige Lösungen der Haarfarbstoffvorprodukte, aber auch mehr oder weniger verdickte Lösungen. Gut handhabbar sind auch Zubereitungen in Form eines Gels oder einer Creme. Als besonders brauchbar hat sich die Anwendung einer wäßrigen Zubereitung in Form eines Schaums erwiesen, wobei dieser Schaum aus einer wäßrigen Lösung mit Hilfe eines unter Druck stehenden Gases erzeugt wird (Aerosol-Schaum).

Die erfindungsgemäß verwendeten Haarfarbstoffvorprodukte liegen in der wäßrigen Zubereitung in gelöster Form vor. Insgesamt enthalten die Zubereitungen üblicherweise nicht mehr als 6 Gew.-% an den genannten Farbstoffvorprodukten. Höhere Konzentrationen sind allenfalls in Sonderfällen sinnvoll. Die Untergrenze wird durch eine ausreichende Färbegeschwindigkeit bestimmt und liegt üblicherweise bei etwa 0,05 Gew.-% an Farbstoffvorprodukten insgesamt. Bevorzugt wird in den Zubereitungen ein Gehalt an Farbstoffvorprodukten von etwa 0,1 Gew.-% bis etwa 4 Gew.-%, insbesondere von etwa 0,2 Gew.-% bis etwa 3 Gew.-%.

Das Mengenverhältnis der Farbstoffvorprodukte bestimmt neben der Qualität des Haares, an dem das Verfahren ausgeführt wird, den Farbton, der mit dem Verfahren erreicht wird. Mit Zubereitungen, die 1,2,4-Trihydroxybenzol, N,N-Bis-(2-hydroxyethyl)-p-phenylendiamin und 2,4,5,6-Tetraaminopyrimidin enthalten, werden je nach Mengenverhältnis mittelblonde bis mittelbraune Farbtöne erzielt. Zubereitungen, die zusätzlich 2,7-Dihydroxynaphthalin enthalten, führen zu Färbungen, die je nach Mengenverhältnis der Vorprodukte von hellblond bis dunkelblond reichen. Bemerkenswert ist, daß es sich in jedem Falle um natürlich wirkende klare Farbtöne handelt, die auch bei mehrfacher Anwendung des Färbeverfahrens nicht stumpf oder grau werden.

Vorzugsweise liegen die Konzentrationen der einzelnen Farbstoffvorprodukte in den wäßrigen Zubereitungen in folgenden Grenzen: 1,2,4-Trihydroxybenzol zwischen etwa 0,01 Gew.-% und etwa 3 Gew.-%, insbesondere zwischen etwa 0,02 Gew.-% und etwa 1 Gew.-%; N,N-Bis-(2-hydroxyethyl)-p-phenylendiamin zwischen etwa 0,01 Gew.-% und etwa 4 Gew.-%, insbesondere zwischen etwa 0,02 Gew.-% und etwa 2 Gew.-%; 2,4,5,6-Tetraaminopyrimidin zwischen etwa 0,01 Gew.-% und etwa 3 Gew.-% und insbesondere zwischen etwa 0,05 Gew.-% und etwa 0,5 Gew.-%. 2,7-Dihydroxynaphthalin, das nur in einer besonderen Ausführungsform der Erfindung in den wäßrigen Zubereitungen verwendet wird, liegt dann vorzugsweise in Konzentrationen zwischen etwa 0,01 Gew.-% und etwa 2 Gew.-%, insbesondere zwischen etwa 0,02 Gew.-% und etwa 0,5 Gew.-% vor. Auch dann, wenn die aminischen Farbstoffvorprodukte bei der Herstellung der Zubereitungen als lösliche Salze, z. B. als Chloride, Sulfate oder Acetate, eingesetzt werden, beziehen sich die vorgenannten Mengen stets auf die freien Basen. Ebenso beziehen sich die Mengenangaben für die phenolischen Vorprodukte auf die freien Phenole auch dann, wenn zur Herstelllung der Zubereitungen hydrolisierbare Derivate der Phenole, wie etwa die Ester mit Essigsäure, verwendet werden.

Selbstverständlich ist es möglich, die im erfindungsgemäßen Verfahren verwendeten wäßrigen Zubereitungen durch Zusatz weiterer Haarfarbstoffvorprodukte oder auch durch direktziehende Farbstoffe, wie sie bei der Haarfärberei üblich sind, zu modifizieren, wenn dadurch die positiven Eigenschaften nicht verloren gehen. Es ist aber ein besonderer Vorzug gerade der im erfindungsgemäßen Verfahren verwendeten Haarfärbezubereitungen, daß allein mit den genannten Haarfarbstoffvorprodukten die gesamte Palette der helleren Naturfarbtöne abgedeckt werden kann, wie sie von vielen Menschen mit ergrautem Haar gewünscht werden. Weitere Farbstoffvorprodukte oder direktziehende Farbstoffe werden deshalb vorzugsweise in Mengen eingesetzt, die insgesamt nicht größer sind als die Gesamtmenge der im Vorstehenden namentlich genannten Farbstoffvorprodukte. Besonders bevorzugt beträgt ihre Menge nicht mehr als 20 und insbesondere nicht mehr als 10 Gew.-% der Gesamtmenge der namentlich genannten Farbstoffvorprodukte. In der besonders bevorzugten Ausführungsform werden außer den namentlich genannten Farbstoffvorprodukten keine weiteren Farbstoffvorprodukte in den erfindungsgemäß verwendeten wäßrigen Zubereitungen verwendet.

Prinzipiell läßt sich eine Färbung von Haar bereits mit wäßrigen Lösungen, die allein die Haarfarbstoffvorprodukte enthalten, durchführen. Üblicherweise enthalten die wäßrigen Zubereitungen aber weitere Hilfs- und Zusatzstoffe, wie sie auch in anderen Oxidationshaarfärbemitteln üblich sind. Als Hilfsstoffe werden dabei solche Substanzen angesehen, die in erster Linie die Färbewirkung unterstützen, beispielsweise dadurch, daß sie die Stabilität und Struktur der Zubereitung gewährleisten, die Löslichkeit der Komponenten sicherstellen und die schnelle und zuverlässige Anwendung der Zubereitung auf dem Haar erleichtern. Als Zusatzstoffe werden dagegen solche Substanzen angesehen, die Sekundäreffekte vermitteln, also beispielsweise die Kämmbarkeit des Haares verbessern, die elektrostatische Aufladung vermindern, dem Haar Glanz oder Fülle vermitteln oder andere haarpflegende Wirkungen entfalten.

Beispiele für Hilfsstoffe sind Tenside aller Art, Komplexbildner, Antioxidantien und Reduktionsmittel, Alkalisierungsmittel, organische Lösungsmittel, andere Lösungsvermittler, Verdickungsmittel sowie gegebenenfalls Treibmittel für Aerosole, die aber nicht als Bestandteile der Zubereitungen gerechnet werden. Als Zusatzstoffe oder Haarpflegemittel seien beispielsweise kationische und zwitterionische Polymere, haarkonditionierende Verbindungen aus der Gruppe der Phospholipide, Silikonöle und Proteinhydrolysate, Parfümöle, Vitamine und ähnliche Wirkstoffe wie Panthenol, Pantothensäure und Pflanzenextrakte sowie Fette und Wachse, einschließlich Fettsäurealkanolamide, genannt.

Als Tenside eignen sich alle bekannten Arten von Tensiden, also anionische, nichtionische, kationische und zwitterionische bzw. amphotere Tenside. Beispiele für geeignete anionische Tenside sind Alkylsulfate und Alkylpolyglykolethersulfate, Alkansulfonate, α-Olefinsulfonate, N-Acylsarkosinate, Ethercarboxylate und Sulfobernsteinsäuremono- und -dialkylester.

Wichtigste nichtionische Tenside sind die Anlagerungsprodukte von Ethylenoxid und/oder Propylenoxid an lineare Fettalkohole oder Oxoalkohole, an Glycerinfettsäureester, an Rizinusöl oder gehärtetes Rizinusöl, an Sorbitanfettsäureester und an Fettsäurealkanolamide. Von Bedeutung sind auch die Alkylglykoside.

Als kationische Tenside eignen sich in erster Linie quartäre Ammoniumverbindungen, insbesondere langkettige Alkyltrimethylammoniumsalze, beispielsweise Cetyltrimethylammoniumbromid, oder auch kationisch modifizierte Silikonöle.

Als zwitterionische Tenside eignen sich insbesondere die Betaine und Sulfobetaine wie beispielsweise Kokosalkyldimethylammoniumglycinat und das unter der INCI-Bezeichnung Cocoamidopropyl-Betain bekannte Fettsäureamidderivat. Beispiele geeigneter ampholytischer Tenside sind Kokosalkylaminopropionat und Fettalkylsarkosinate.

Selbstverständlich können in den erfindungsgemäß verwendeten Zubereitungen mehrere Tenside auch unterschiedlicher Art nebeneinander verwendet werden. Art und Menge richten sich nach dem gewünschten Effekt, wobei die Tenside vor allem als Netzmittel, als Emulgatoren oder als Schaumbildner wirken.

Komplexbildner können den erfindungsgemäß verwendeten Zubereitungen zugesetzt werden, um Störungen durch Wasserhärte und insbesondere durch Schwermetallionen auszuschließen. Besonders geeignet sind Aminopolycarbonsäuren, Polyphosphonsäuren und Polycarbonsäuren, wie beispielsweise Ethylendiamintetraessigsäure, Nitrilotriessigsäure, Ethylendiamintetramethylenphosphonsäure, Diethylentriaminpentamethylenphosphonsäure, 1-Hydroxy-ethan-1,1-diphosphonsäure und Zitronensäure. Besonders bevorzugt werden Ethylendiamintetraessigsäure und Hydroxyethandiphosphonsäure. Zur Herstellung der Zubereitungen werden die Komplexbildner in den meisten Fällen als wasserlösliche Salze, insbesondere Alkalisalze, eingesetzt. Ihr Gehalt in den Zubereitungen liegt vorzugsweise zwischen etwa 0,01 und etwa 1 Gew.-%, insbesondere zwischen etwa 0,05 und etwa 0,2 Gew.-%, gerechnet als freie Säuren.

Reduktionsmittel und Antioxidantien können in den erfindungsgemäßen Zubereitungen dazu dienen, eine vorzeitige Oxidation der Farbstoffvorprodukte während der Herstellung und Lagerung der Zubereitungen zu verhindern. Beispiele dieser Hilfsstoffe sind Natriumsulfit, Natriumhydrogensulfit, Natirumdithionit, Ascorbinsäure und Isoascorbinsäure. Die Menge an derartigen Hilfsstoffen liegt vorzugsweise zwischen etwa 0,01 und etwa 1 Gew.-%, insbesondere zwischen etwa 0,05 und etwa 0,1 Gew.-%, bezogen auf die Zubereitung als Ganzes.

Unter Alkalisierungsmitteln werden solche Stoffe verstanden, die eine gewisse Pufferkapazität gegen Säuren besitzen. Beispiele sind Alkanolamine, insbesondere Monoethanolamin, Carbonate, Hydrogencarbonate, insbesondere Ammoniumhydrogencarbonat, Guanidine und Phosphate. Neben der Einstellung des pH-Wertes bewirken sie in der Regel auch eine Quellung der Haare und damit eine leichtere Anfärbbarkeit. Die Menge an Alkalisierungsmitteln liegt in den Zubereitungen vorzugsweise zwischen etwa 1 und etwa 8 Gew.-%, insbesondere zwischen etwa 2 und etwa 4 Gew.-%. Wie bei den anderen Hilfsstoffen können auch bei den Alkalisierungsmitteln mehrere Substanzen nebeneinander verwendet werden. Der pH-Wert der Zubereitungen soll vorzugsweise zwischen etwa 6,5 und etwa 10,5, insbesondere zwischen etwa 8 und etwa 9,5 liegen.

Organische Lösungsmittel können dazu dienen, Farbstoffvorprodukte und gegebenenfalls weiterere Inhaltsstoffe in den wäßrigen Zubereitungen auch unter schwierigen Lagerbedingungen in Lösung zu halten. Die Lösungsmittel müssen dazu selbst eine ausreichende Wasserlöslichkeit besitzen oder, vorzugsweise, vollständig wassermischbar sein. Beispiele geeigneter Lösungsmittel sind Ethanol, Propanol, iso-Propanol, tert.-Butanol, Ethylenglykol, Propylenglykol, Diethylenglykol, Dipropylenglykol, Glycerin sowie Ether und Ester der genannten Glykole. Besonders bevorzugt werden Ethanol und Propylenglykol. Der Gehalt an organischen Lösungsmitteln in den erfindungsgemäß verwendeten Zubereitungen kann bis etwa 40 Gew.-% betragen, in Sonderfällen aber auch höher liegen. Vorzugsweise liegt der Gehalt an organischen Lösungsmitteln zwischen etwa 0,5 Gew.-% und etwa 30 Gew.-%, insbesondere zwischen etwa 1 Gew.-% und etwa 20 Gew.-%.

Beispiele geeigneter Verdickungsmittel sind vor allem die Cellulosederivate Methylcellulose, Hydroxyalkylcellulose und Carboxymethylcellulose. Geeignet sind aber auch vollsynthetische Polymere wie z.B. Polyvinylalkohol, Vinylacetat-Homo- und Copolymere, N-Vinylpyrrolidon-Homo- und Copolymere und insbesondere Acrylsäure-Homo- und Copolymere.

Als Hilfsstoffe sind auch die Treibgase anzusehen, die bei der bevorzugten Anwendung der wäßrigen Zubereitungen in Form eines Aerosol-Schaums zusammen mit den wäßrigen Zubereitungen in einem Behälter verpackt werden. Als Treibgase eignen sich insbesondere Propan-Butan-Gemische, Dimethylether, Distickstoffoxid, Kohlendioxid und prinzipiell auch die früher üblichen Fluorchlorkohlenwasserstoffe. Vorzugsweise werden Propan-Butan-Gemische verwendet, von denen etwa 4 bis 10 Gew.-%, bezogen auf 100 Gew.-% wäßrige Zubereitung in den Behältern eingesetzt werden.

Als wichtige Zusatzstoffe, die in den erfindungsgemäß verwendeten Zubereitungen eingesetzt werden können, seien vor allem die Haarpflegemittel und hier insbesondere die ionischen Polymeren erwähnt. Besondere Bedeutung besitzen die kationisch modifizierten Polymeren, die Glanz, Fülle und Kämmbarkeit der Haare verbessern. Beispiele sind quaternierte Celluloseether, Polysiloxane mit quartären Gruppen, Acrylamid-Dimethyldialkylammoniumchlorid-Copolymere, quaternierte Methylaminoethylmethacrylat-Vinylpyrrolidon-Copolymere und derivatisierter Polyvinylalkohol mit quartären Ammoniumgruppen. Ein im Rahmen der Erfindung besonders bevorzugter Zusatzstoff dieser Art ist das unter der INCl-Bezeichnung Polyquaternium-10 geführte quaternierte Cellulosederivat. Weitere erwähnenswerte Zusatzstoffe sind Proteinhydrolysate, Kondensationsprodukte von Proteinhydrolysaten mit Fettsäure und Proteinhydrolysate mit quartären Ammoniumgruppen.

Beispiele weiterer Hilfs- und Zusatzstoffe, die auch in den erfindungsgemäß verwendeten Zubereitungen eingesetzt werden können, finden sich in den Handbüchern der Kosmetik, beispielsweise aber auch als Auflistung in der oben erwähnten deutschen Offenlegungsschrift 19717223. Es ist selbstverständlich, bei der Auswahl der Hilfs- und Zusatzstoffe darauf zu achten, daß nur physiologisch verträgliche Substanzen eingesetzt werden, die das färberische Ergebnis der erfindungsgemäß eingesetzten Haarfarbstoffvorprodukt-Kombinationen nicht beeinträchtigen. In welchen Mengen die einzelnen Hilfs- und Zusatzstoffe eingesetzt werden, richtet sich nach dem Effekt, der damit erreicht werden soll.

Die Herstellung der erfindungsgemäß verwendeten wäßrigen Zubereitungen bietet keine besonderen Probleme. Besonders zweckmäßig ist es, die Haarfarbstoffvorprodukte in einem Teil des Wassers unter Zusatz von Alkalisierungsmittel, gegebenenfalls unter leichtem Erwärmen, bis zur vollständigen Teilchenfreiheit zu lösen. Anstelle der freien aminischen Basen N,N-Bis-(2-hydroxyethyl)-p-phenylen-diamin und 2,4,5,6-Tetraaminopyrimidin kann auch von wasserlöslichen Salzen dieser Verbindungen ausgegangen werden, beispielsweise von den Sulfaten. Anstelle der Phenole kann auch von Estern dieser Phenole, insbesondere vom Triacetat des 1,2,4-Trihydroxybenzols ausgegangen werden, sofern sichergestellt ist, daß bis zum Einsatz der Zubereitungen weitgehende oder vollständige Hydrolyse zu den freien Phenolen eingetreten ist. Getrennt von den Farbstoffvorprodukten können dann die übrigen Hilfs- und Zusatzstoffe in Wasser zu einer klaren Lösung gebracht werden, wobei es zweckmäßig sein kann, wenig wasserlösliche Substanzen, wie beispielsweise Parfümöl, zunächst in organischen Lösungsmitteln, gegebenenfalls unter Zusatz weiterer Lösungsvermittler, aufzunehmen und dann in dieser Form der wäßrigen Lösung der übrigen Hilfs- und Zusatzstoffe zuzusetzen. Die getrennt hergestellte wäßrige Lösung der Farbstoffvorprodukte wird dann als letztes eingemischt. Es ist aber ohne weiteres möglich, die Mischungsreihenfolge ganz oder teilweise umzustellen. Um einer vorzeitigen Oxidation durch Luftsauerstoff vorzubeugen, hat es sich als zweckmäßig erwiesen, in einzelnen Stufen oder insgesamt unter Schutzgas, beispielsweise Stickstoff, zu arbeiten, bis die fertige Zubereitung in die vorgesehenen Behälter abgefüllt ist. Wenn die Anwendung der Zubereitung als Aerosol vorgesehen ist, wird als letztes das Treibgas in die Behälter eingefüllt.

Die Anwendung der Zubereitungen im erfindungsgemäßen Verfahren erfolgt bei der Färbung von menschlichem Haar in der Weise, daß etwa 2 bis etwa 20 g, vorzugsweise etwa 5 bis etwa 10 g der Zubereitung auf dem Kopfhaar gleichmäßig verteilt werden und dort vorzugsweise etwa 5 bis etwa 60 Minuten, insbesondere etwa 10 bis etwa 30 Minuten verbleiben. In dieser Zeit entwickelt sich unter Mitwirkung des Luftsauerstoffs die eigentliche Färbung auf dem Haar. Eine Erwärmung ist nicht notwendig. Die wäßrigen Zubereitungen können auf trockenes Haar aufgetragen werden oder aber, wenn das Haar vorher gewaschen wurde, auch auf das feuchte Haar. Kleinere Mengen der wäßrigen Zubereitungen können nach dem Färbevorgang auf dem Haar verbleiben. In der Regel werden aber die überschüssigen Mengen an wäßriger Zubereitung nach der Einwirkzeit aus dem Haar ausgespült. Dies kann ohne weiteres mit Wasser geschehen oder aber auch im Rahmen einer erneuten Haarwäsche mit einer Shampoo-Lösung. Bei der Anwendung hat sich der Einsatz der wäßrigen Zubereitung in Form von Aerosol-Schaum besonders bewährt, da hier große Volumina an Haarfärbemittel erzeugt werden, deren gleichmäßige Verteilung auf dem Haar besonders einfach ist.

Selbstverständlich ist es auch möglich, das Verfahren nur auf Teile des Kopfhaares, beispielsweise im Schläfenbereich, anzuwenden und die übrigen Kopfhaare unbehandelt zu lassen. Es wird dann entsprechend weniger an Farbstoffzubereitung eingesetzt. Kleinere Mengen kommen auch dann zum Einsatz, wenn andere Haarpartien, wie beispielsweise Bärte, gefärbt werden sollen.

In prinzipiell gleicher Weise ist die Färbung von nichtmenschlichem Haar, beispielsweise von Wolle oder Pelzen oder von anderen keratinhaltigen Materialien möglich. Da es hier auf physiologische Verträglichkeit der Hilfs- und Zusatzstoffe in den wäßrigen Zubereitungen weniger ankommt, können auch solche Hilfs- und Zusatzstoffe verwendet werden, die im kosmetischen Bereich nicht zum Einsatz kommen.

### Beispiele

In den im folgenden beschriebenen Zubereitungen sind einige Handelsprodukte enthalten, die zunächst erläutert werden sollen:
- Antil® HS 60: (Betaintensid der Firma Th. Goldschmidt, INCI-Bezeichnung Cocamidopropyl betaine + Glyceryl laurate)
- Brij® 35: (nichtionisches Tensid der Firma ICI, INCI-Bezeichnung Laureth-23)
- Carbopol® 1382: (vernetztes Acrylsäure-Copolymer der Firma Goodrich, INCI-Bezeichnung Acrylates/C10-30 alkyl acrylate crosspolymer)
- Dehyton® K: (zwitterionisches Tensid der Firma Henkel, INCI-Bezeichnung Cocamidopropyl betaine)
- Eumulgin® B2: (nichtionischer Emulgator der Firma Henkel, INCI-Bezeichnung Ceteareth-20)
- Lanette® O: (Cetylstearylalkohol der Firma Henkel, INCI-Bezeichnung Cetearyl alcohol)
- Plantacare® 1200: (Alkylpolyglukosid der Firma Henkel, INCI-Bezeichnung Lauryl glucoside)

### 1. Eine wäßrige Zubereitung von Haarfarbstoffvorprodukten hatte folgende Zusammensetzung (Zubereitung 1):

| **Inhaltsstoffe** | **Konzentration/Gew.-%** |
|---|---|
| Brij35 | 0,05 |
| Antil HS 60 | 2,00 |
| Ammoniumhydrogencarbonat | 2,00 |
| Ethanol (96 %) | 20,00 |
| Parfüm | 0,10 |
| Natriumsulfit | 0,10 |
| Monoethanolamin | 1,00 |
| 2,4,5,6-Tetraaminopyrimidin Sulfat | 0,26 |
| 1,2,4-Trihydroxybenzol | 0,15 |
| N,N-Bis-(2-hydroxyethyl)-p-phenylendiamin Sulfat | 0,097 |
| Wasser | zu 100,00 |

Die Zubereitung wurde in mehreren Stufen hergestellt:
a) In der Hälfte der Wassermenge wurden Natriumsulfit, Ammoniumhydrogencarbonat und die beiden Tenside gelöst.
b) Das Parfümöl wurde im Ethanol gelöst und in dieser Form zu der unter a) hergestellten wäßrigen Lösung gegeben.
c) Im restlichen Wasser wurden unter Stickstoffatmosphäre zunächst das Monoethanolamin und dann unter leichtem Erwärmen die drei Farbstoffvorprodukte bis zur vollständigen Teilchenfreiheit gelöst. Die Lösung der Farbstoffvorprodukte wurde dann in die Lösung der übrigen Produkte, die vorher ebenfalls mit Stickstoff überschichtet worden war, eingerührt. Die fertige wäßrige Zubereitung wies einen pH-Wert von 8,9 auf. Sie wurde in Aerosolbehälter abgefüllt, die mit einem Schaumsprayventil ausgerüstet waren, und dort mit einem Gemisch aus Propan und Butan als Treibgas versetzt. Auf 94 g der wäßrigen Zubereitung wurden 6 g Treibgas verwendet.

### 2. In gleicher Weise wie in Beispiel 1 wurde eine wäßrige Zubereitung von Farbstoffvorprodukten hergestellt, die die in der folgenden Tabelle beschriebene Zusammensetzung hatte (Zubereitung 2):

| **Inhaltsstoffe** | **Konzentration/Gew.-%** |
|---|---|
| Brij 35 | 0,05 |
| Antil HS 60 | 2,00 |
| Ammoniumhydrogencarbonat | 2,00 |
| Ethanol (96 %) | 20,00 |
| Parfüm | 0,10 |
| Natriumsulfit | 0,10 |
| Monoethanolamin | 1,00 |
| 2,7-Dihydroxynaphthalin | 0,08 |
| 2,4,5,6-Tetraaminopyrimidin Sulfat | 0,17 |
| 1,2,4-Trihydroxybenzol | 0,04 |
| N,N-Bis-(2-hdyroxyethyl)-p-phenylendiamin Sulfat | 0,06 |
| Wasser | zu 100,00 |

Auch diese wäßrige Zubereitung wurde in gleicher Weise wie in Beispiel 1 in Aerosoldosen abgefüllt.

### 3. Färbeversuche

Ein Teil der Färbeversuche wurde an weißem Standardwollgewebe der Firma. WFK, Krefeld, (Bezeichnung 60A) durchgeführt. Dazu wurden jeweils 2 g der aufgeschäumten wäßrigen Zubereitung der Farbstoffvorprodukte in Probestücke des Gewebes mit den Abmessungen 9 x 5 cm gleichmäßig eingearbeitet und bei Raumtemperatur an der Luft 20 Minuten liegengelassen. Nach dieser Zeit wurde das überschüssige Haarfärbemittel mit lauwarmem Leitungswasser gründlich ausgespült. Die Wollproben wurden dann mit einem Föhn getrocknet und 24 Stunden liegengelassen, bevor ihre Färbung mit einem Spektralphotometer des Typs CM-2002 der Firma Minolta vermessen wurde. Die Auswertung erfolgte nach dem CIE-LAB-System, bei dem folgende Kenngrößen verwendet werden:
L*: Helligkeit (0 = dunkel, 100 = hell)
a*: Rotgrünwert (negativ = grün, positiv = rot)
b*: Gelbblauwert (negativ = blau, positiv = gelb)

Mit Zubereitung 1 wurden folgende Ergebnisse erhalten:

| **Anwendung** | **L*** | **a*** | **b*** |
|---|---|---|---|
| 1mal | 66,1 | +2,6 | +8,4 |
| 3mal | 52,5 | +3,3 | +7,8 |
| 5mal | 45,3 | +2,6 | +7,6 |

Die Farbwerte zeigen deutlich einen hellen Braunton an, dessen Farbtiefe bei mehrmaliger Anwendung ansteigt, ohne daß es zu einer wesentlichen Farbverschiebung kommt.

Mit Zubereitung 2 wurden folgende Meßwerte erhalten:

| **Anwendung** | **L*** | **a*** | **b*** |
|---|---|---|---|
| 1mal | 71,8 | +1,5 | +14,9 |
| 3mal | 61,4 | +2,8 | +12,5 |

Hier zeigen die Farbwerte einen hellen Gelbton an.

Zur Beurteilung der Wirkung auf graumeliertem Haar wurden in gleicher Weise wie auf Wolle Färbeversuche an Büscheln von graumeliertem Büffelbauchhaar durchgeführt. Mit Zubereitung 1 wurde hier nach mehrmaliger Anwendung eine gute Grauabdeckung erreicht und ein natürlich wirkender mittelbrauner Farbton erzielt. Auch mit Zubereitung 2 wurde nach wiederholter Anwendung eine gute Grauabdeckung erzielt mit einem Farbton, der als goldbeige bezeichnet werden kann.

Zur Beurteilung der Wirkung auf grauem menschlichen Haar wurden Versuche an weiblichen Probanden mit Zubereitungen durchgeführt, die in ihren Zusammensetzungen im wesentlichen mit denen der Zubereitungen in den Beispielen 1 bzw. 2 übereinstimmten. Auch diese Zubereitungen wurden in Form von Aerosol-Schaum angewendet.

Bei den Probanden, die die Zubereitung ähnlich Beispiel 1 erprobten, handelte es sich um zehn Frauen im Alter von 48 bis 79 Jahren, deren Haarfarbe ursprünglich dunkelblond bis hellbraun gewesen war, die aber bei Versuchsbeginn einen mittleren bis starken Grauanteil im Kopfhaar aufwiesen. Vier der Probanden hatten dauergewelltes Haar.

Bei den Probanden, die eine Zubereitung ähnlich Beispiel 2 erprobten, handelte es sich um zehn Frauen im Alter von 47 bis 67 Jahren, die ursprünglich eine mittelblonde bis hellblonde Haarfarbe hatten, und deren Haar bei Versuchsbeginn ebenfalls einen mittleren bis starken Grauanteil aufwies. Von diesen Probanden hatten sieben dauergewelltes Haar.

Die Anwendung der Zubereitungen war bei beiden Kollektiven gleich: 5 bis 10 g der jeweiligen Zubereitung wurden nach dem Waschen in das handtuchfeuchte Haar gleichmäßig eingearbeitet und dort 20 Minuten belassen. Danach wurden die Haare mit Wasser ausgespült und mit einem Föhn getrocknet. Die Anwendung erfolgte vier- bis sechsmal in einem Zeitraum von zwei Wochen.

Bei allen Probanden wurde nach vier oder fünf Anwendungen der gewünschte ursprüngliche Naturfarbton bei guter Grauabdeckung erreicht. Kontrolluntersuchungen nach vier Wochen ließen keinerlei Veränderung des erreichten Farbtons erkennen.

### 4. Gelförmige Haarfärbemittel

In der folgenden Tabelle ist die Rahmenrezeptur einer gelförmigen wäßrigen Zubereitung, die sich für das erfindungsgemäße Verfahren eignet, angegeben.

| **Inhaltsstoffe** | **Konzentration/Gew.-%** |
|---|---|
| Dehyton K | 1,0 |
| Carbopol 1382 | 0,5 |
| Monoethanolamin | 1,0 |
| Natriumsulfit | 0,2 |
| Propylenglykol | 1,0 |
| NaOH (10 %) | nach Bedarf |
| Farbstoffvorprodukte* | 0,2 bis 2 |
| Wasser | zu 100 |

| | |
|---|---|
| * Gemisch der Farbstoffvorprodukte 1,2,4-Trihydroxybenzol, N,N-Bis-(2- hydroxyethyl)-p-phenylendiamin, 2,4,5,6-Tetraaminopyrimidin und gegebenenfalls 2,7-Dihydroxynaphthalin | |

Das Gel kann folgendermaßen hergestellt werden:
Zunächst wird das Acrylsäure-Copolymerisat in der Hälfte der Wassermenge dispergiert und nach vollständiger Quellung mit Natronlauge auf einen pH-Wert von etwa 5 eingestellt. Dann werden das zwitterionische Tensid und Natriumsulfit, gelöst in einem weiteren Viertel der Wassermenge, zugesetzt. In der restlichen Wassermenge werden Monoethanolamin, Propylenglykol und die Farbstoffvorprodukte bis zur Teilchenfreiheit gelöst, bevor diese Lösung dann ebenfalls dem Gel zugegeben wird. Abschließend wird der pH-Wert mit Natronlauge auf etwa 9 eingestellt.

### 5. Cremeförmige Haarfärbemittel

Die Rahmenrezeptur einer cremeförmigen wäßrigen Zubereitung der Farbstoffvorprodukte, die sich für das erfindungsgemäße Verfahren eignet, ist in der folgenden Tabelle angegeben.

| **Inhaltsstoffe** | **Konzentration/Gew.-%** |
|---|---|
| Lanette O | 16,0 |
| Eumulgin B2 | 1,0 |
| Natriumsulfit | 0,2 |
| Monoethanolamin | 1,0 |
| Ammoniumhydrogencarbonat | 2,0 |
| Dehyton K | 1,0 |
| Plantacare 1200 | 2,0 |
| Farbstoffvorprodukte* | 0,2 bis 2 |
| Wasser | zu 100 |

| | |
|---|---|
| * Gemisch der Farbstoffvorprodukte 1,2,4-Trihydroxybenzol, N,N-Bis-(2- hydroxyethyl)-p-phenylendiamin, 2,4,5,6-Tetraaminopyrimidin und gegebenenfalls 2,7-Dihydroxynaphthalin | |

Die Creme kann auf folgendem Wege hergestellt werden:
Cetylstearylalkohol und nichtionischer Emulgator werden geschmolzen, und in die Schmelze wird dann die Hälfte der Wassermenge, die zuvor auf 80° C erhitzt worden ist, unter Rühren einemulgiert. Nach dem Abkühlen werden das zwitterionische Tensid, das Alkylpolyglukosid und Natriumsulfit zugegeben. In der restlichen Wassermenge werden die Farbstoffvorprodukte und Ammoniumhydrogencarbonat bis zur Teilchenfreiheit gelöst, bevor diese Lösung dann zusammen mit Monoethanolamin ebenfalls der Emulsion zugesetzt wird. Sofern nötig, wird abschließend der pH-Wert auf etwa 9 eingestellt.

## Patentansprüche

1. Verfahren zur Färbung von Haar, bei dem eine wäßrige Zubereitung von Farbstoffvorprodukten ohne Zusatz von Oxidationsmittel mit dem Haar in Kontakt gebracht wird und sich die Farbe durch Einwirkung des Sauerstoffs der Luft entwickelt, dadurch gekennzeichnet, daß die wäßrige Zubereitung als Farbstoffvorprodukte 1,2,4-Trihydroxybenzol, N,N-Bis-(2-hydroxyethyl)-p-phenylendiamin und 2,4,5,6-Tetraaminopyrimidin enthält.

2. Verfahren nach Anspruch 1, bei dem die wäßrige Zubereitung 5 bis 60 Minuten, vorzugsweise 10 bis 30 Minuten mit dem Haar in Gegenwart von Luftsauerstoff in Kontakt bleibt und dann mit Wasser oder einer Shampoo-Lösung aus dem Haar ausgespült wird.

3. Verfahren nach einem der Ansprüche 1 oder 2, bei dem die wäßrige Zubereitung als weiteres Farbstoffvorprodukt 2,7-Dihydroxynaphthalin enthält.

4. Verfahren nach einem der Ansprüche 1 bis 3, bei dem die wäßrige Zubereitung in Form eines Schaums auf das Haar aufgetragen wird, der mit Hilfe eines unter Druck stehenden Gases erzeugt wird.

5. Wäßrige Zubereitung von Haarfarbstoffvorprodukten zur Durchführung eines Verfahrens gemäß einem der Ansprüche 1 bis 4, enthaltend 1,2,4-Trihydroxybenzol, N,N-Bis-(2-hydroxyethyl)-p-phenylendiamin und 2,4,5,6-Tetraaminopyrimidin.

6. Wäßrige Zubereitung nach Anspruch 5, die als weiteres Farbstoffvorprodukt 2,7-Dihydroxynaphthalin enthält.

7. Wäßrige Zubereitung nach einem der Ansprüche 5 oder 6, die einen pH-Wert im Bereich von 6,5 bis 10,5, vorzugsweise im Bereich von 8 bis 9,5 aufweist und weiterhin wenigstens einen weiteren Hilfs- oder Zusatzstoff aus der Gruppe Tenside, Komplexbildner, Antioxidantien und Reduktionsmittel, Alkalisierungsmittel, Lösungsvermittler, organische Lösungsmittel, Verdickungsmittel, Parfüm und Haarpflegemittel enthält.

8. Wäßrige Zubereitung nach einem der Ansprüche 5 bis 7, enthaltend
0,01 bis 3 Gew.-%, vorzugsweise 0,02 bis 1 Gew.-% 1,2,4-Trihydroxybenzol,
0,01 bis 4 Gew.-%, vorzugsweise 0,02 bis 2 Gew.-% N,N-Bis-(2-hydroxyethyl)-p-phenylendiamin,
0,01 bis 3 Gew.-%, vorzugsweise 0,05 bis 0,5 Gew.-% 2,4,5,6-Tetraaminopyrimidin,
0,01 bis 1 Gew.-%, vorzugsweise 0,05 bis 0,2 Gew.-% Komplexbildner, insbesondere aus der Gruppe Ethylendiamintetraessigsäure, Hydroxyethandiphosphonsäure und deren Mischungen,
0,01 bis 1 Gew.-%, vorzugsweise 0,05 bis 0,1 Gew.-% Reduktionsmittel und Antioxidantien, insbesondere aus der Gruppe Natriumsulfit, Natriumdithionit, Ascorbinsäure, Isoascorbinsäure und deren Mischungen,
1 bis 8 Gew.-%, vorzugsweise 2 bis 4 Gew.-% Alkalisierungsmittel, insbesondere aus der Gruppe Ammoniumhydrogencarbonat, Monoethanolamin und deren Mischungen,
0 bis 40 Gew.-%, vorzugsweise 0,5 bis 30 Gew.-% weitere Hilfs- und Zusatzstoffe aus der Gruppe Tenside, Verdickungsmittel, Lösungsvermittler, organische Lösungsmittel, Parfüm, Haarpflegemittel und deren Mischungen und
Rest zu 100 Gew.-% Wasser.

9. Wäßrige Zubereitung nach Anspruch 8, enthaltend 0,01 bis 2 Gew.-%, vorzugsweise 0,02 bis 0,5 Gew.-% 2,7-Dihydroxynaphthalin.

10. Wäßrige Zubereitung nach einem der Ansprüche 5 bis 9, verpackt zusammen mit Treibgas in einem Aerosolbehälter, wobei die wäßrige Zubereitung soviel Tensid enthält, daß die wäßrige Zubereitung bei Entnahme aus dem Behälter als Schaum austritt.

## Claims

1. Hair dyeing method in which an aqueous preparation of dye precursors is brought into contact with hair without addition of any oxidizing agent, and in which the colour is obtained as a result of the effect of atmospheric oxygen, the method being characterized by the fact that the aqueous preparation contains as dye precursors 1,2,4-trihydroxybenzene, N,N-bis-(2-hydroxyethyl)-p-phenylenediamine and 2,4,5,6-tetraaminopyrimidine.

2. Method as claimed in Claim 1, characterized in that the aqueous preparation remains in contact with the hair for 5 to 60 minutes, preferably 10 to 30 minutes, in the presence of atmospheric oxygen, and is then rinsed with water or a shampoo solution.

3. Method as claimed in one of the Claims 1 or 2, in which the aqueous preparation contains 2,7-dihydroxynaphthalene as an additional dye precursor.

4. Method as claimed in one of the Claims 1 to 3, characterized in that the aqueous preparation is applied to the hair in the form of foam produced with the aid of a pressurized gas.

5. Aqueous preparation of hair dye precursors for a method according to one of the Claims 1 to 4, containing 1,2,4-trihydroxybenzene, N,N-bis-(2-hydroxyethyl)-p-phenylenediamine and 2,4,5,6-tetraaminopyrimidine.

6. Aqueous preparation according to Claim 5, containing 2,7-dihydroxynaphthalene as an additional dye precursor.

7. Aqueous preparation as claimed in Claims 5 or 6, having a pH value in the range from 6.5 to 10.5, preferably from 8 to 9.5, and containing at least one additional auxiliary or additive from the following groups: surfactants, complexing agents, antioxidants and reducing agents, alkalinization agents, solubilizers, organic solvents, thickening substances, perfume and hair grooming products.

8. Aqueous preparation according to one of the Claims 5 to 7, containing
0.01 to 3% by weight, preferably 0.02 to 1% by weight, of 1,2,4-trihydroxybenzene,
0.01 to 4% by weight, preferably 0.02 to 2% by weight, of N,N-bis-(2-hydroxyethyl)-p-phenylenediamine,
0.01 to 3% by weight, preferably 0.05 to 0.5% by weight, of 2,4,5,6-tetraaminopyrimidine,
0.01 to 1% by weight, preferably 0.05 to 0.2% by weight, of complexing agents, in particular from the group of ethylenediaminetetraacetic acid, hydroxyethanediphosphonic acid and blends thereof,
0.01 to 1% by weight, preferably 0.05 to 0.1% by weight, of reducing agents and antioxidants, in particular from the group of sodium sulfite, sodium dithionite, ascorbic acid, isoascorbic acid and blends thereof,
1 to 8% by weight, preferably 2 to 4% by weight, of alkalinization agents, in particular from the group of ammonium hydrogen carbonate, monoethanolamine and blends thereof,
0 to 40% by weight, preferably 0.5 to 30% by weight, of further auxiliaries and additives from the group of surfactants, thickening substances, solubilizers, organic solvents, perfume, hair grooming products, and blends thereof, as well as the rest being water up to 100% by weight.

9. Aqueous preparation according to Claim 8, containing 0.01 to 2% by weight, preferably 0.02 to 0.5% by weight, of 2,7-dihydroxynaphthalene.

10. Aqueous preparation as claimed in one of the Claims 5 to 9, packed into an aerosol container together with propellant gas, with the aqueous preparation containing surfactants in such an amount that the aqueous solution leaves the container in the form of foam.

## Revendications

1. Procédé de teinture de cheveux selon lequel une composition aqueuse de précurseurs de colorant est mise en contact avec les cheveux sans addition de tout agent oxydant, la couleur se développant sous l'influence de l'oxygène de l'air, caractérisé par le fait que la composition aqueuse contient les précurseurs de colorant suivants: le 1,2,4-trihydroxybenzène, la N,N-bis-(2-hydroxéthyle)-p-phénylènediamine et la 2,4,5,6-tétraaminopyrimidine.

2. Procédé selon revendication 1, selon lequel la composition aqueuse est maintenue en contact avec les cheveux en présence de l'oxygène de l'air pendant une durée comprise entre 5 et 60 minutes et de préférence entre 10 et 30 minutes, pour ensuite être rincée avec de l'eau ou une solution de shampooing.

3. Procédé selon l'une des revendications 1 ou 2, caractérisé en ce que la composition aqueuse contient comme précurseur de colorant additionnel le 2,7-dihydroxynaphtalène.

4. Procédé selon l'une des revendications 1 à 3, caractérisé en ce que la composition aqueuse est appliquée sur les cheveux sous forme d'une mousse produite à l'aide d'un gaz sous pression.

5. Composition aqueuse de précurseurs de colorants de cheveux pour réaliser un procédé selon l'une des revendications 1 à 4, contenant le 1,2,4-trihydroxbenzène, la N,N-bis-(2-hydroxyéthyle)-p-phénylènediamine et la 2,4,5,6-tétraaminopyrimidine.

6. Composition aqueuse selon revendication 5, contenant comme précurseur de colorant de cheveux additionnel le 2,7-dihydroxynaphtalène.

7. Composition aqueuse selon l'une des revendications 5 ou 6, dont le pH est compris entre 6,5 et 10,5 et de préférence entre 8 et 9,5, contenant, en outre, au moins un autre auxiliaire ou additif du groupe des tensioactifs, des complexants, des antioxygènes et réducteurs, des agents donnant des propriétés alcalines, des solubiliseurs, des solvants organiques, des épaississants, du parfum et des produits pour les soins capillaires.

8. Composition aqueuse selon l'une des revendications 5 à 7, contenant
0,01 à 3% en poids, de préférence 0,02 à 1% en poids, de 1,2,4-trihydroxybenzène,
0,01 à 4% en poids, de préférence 0,02 à 2% en poids, de N,N-bis-(2-hydroxyéthyle)-p-phénylènediamine,
0,01 à 3% en poids, de préférence 0,05 à 0,5% en poids, de 2,4,5,6-tétraaminopyrimidine,
0,01 à 1% en poids, de préférence 0,05 à 0,2% en poids, de complexants, en particulier du groupe de l'acide éthylènediaminetétraacétique, de l'acide hydroxyéthane diphosphonique et de leurs mélanges,
0,01 à 1% en poids, de préférence 0,05 à 0,1% en poids, de réducteurs et d'antioxygènes, en particulier du groupe du sulfite de sodium, du dithionite de sodium, de l'acide ascorbique et de l'acide isoascorbique et de leurs mélanges,
1 à 8% en poids, de préférence 2 à 4% en poids, d'agents donnant des propriétés alcalines, en particulier du groupe de l'hydrogénocarbonate d'ammonium, du monoéthanolamine et de leurs mélanges,
0 à 40% en poids, de préférence 0,5 à 30% en poids, d'autres auxiliaires et additifs du groupe des tensioactifs, des épaississants, des solubiliseurs, des solvants organiques, du parfum, des produits pour les soins capillaires et de leurs mélanges, et
le reste étant de l'eau à 100% en poids.

9. Composition aqueuse selon revendication 8, contenant 0,01 à 2% en poids, de préférence 0,02 à 0,5% en poids, de 2,7-dihydroxynaphtalène.

10. Composition aqueuse selon l'une des revendications 5 à 9, mise dans un emballage aérosol et accompagnée d'un gaz propulseur, caractérisée par le fait que la composition aqueuse contenant autant de tensioactifs qu'elle forme une mousse en sortant de l'emballage.
